# EUROPEAN PATENT APPLICATION

(11) **EP 4 681 687 A2**
(43) Date of publication of application: **21.01.2026**
(21) Application number: 25205597.5
(22) Date of filing: 19.02.2021
(51) Int. Cl.: A61F 9/007

(54) **BI-MODAL INTRAOCULAR SHUNT INSERTER**

(30) Priority: 20.02.2020 US 202062979353 P
(62) Divisional of application: 24169164.1
(71) Applicant: Aquesys, Inc., North Chicago, IL 60064 (US)
(72) Inventor: ROMODA, Laszlo O., San Clemente, 92673 (US)
(74) Representative: Hoffmann Eitle

(57) **Abstract**

An intraocular shunt inserter for treating glaucoma comprises a needle, a plunger assembly, and a component driver. The needle defines a lumen and a proximal end portion of the needle. The plunger assembly comprises a plunger at least partially disposed within the lumen of the needle, wherein the plunger is configured to advance a shunt through the lumen of the needle upon release. The component driver comprises an actuation member, wherein the component driver elongates from a first length to a second length upon movement of the actuation member for changing a longitudinal relative position of a distal end portion of the plunger, when coupled to a proximal end portion of the plunger, or a distal end portion of the needle, when coupled to a proximal end portion of the needle.

## Description

### CROSS-REFERENCE TO RELATED APPLICATIONS

This application claims the benefit of U.S. Provisional Application No. 62/979,353, filed February 20, 2020, the entirety of which is incorporated herein by reference.

### BACKGROUND

### Field of the Inventions

The present inventions relate to intraocular shunt inserters and their use in methods of implanting an intraocular shunt into an eye.

### Description of the Related Art

Glaucoma is a disease of the eye that affects millions of people. Glaucoma is associated with an increase in intraocular pressure resulting either from a failure of a drainage system of an eye to adequately remove aqueous humor from an anterior chamber of the eye or overproduction of aqueous humor by a ciliary body in the eye. Build-up of aqueous humor and resulting intraocular pressure may result in irreversible damage to the optic nerve and the retina, which may lead to irreversible retinal damage and blindness.

Glaucoma may be treated in a number of different ways. One manner of treatment involves delivery of drugs such as beta-blockers or prostaglandins to the eye to either reduce production of aqueous humor or increase flow of aqueous humor from an anterior chamber of the eye. Glaucoma filtration surgery is a surgical procedure typically used to treat glaucoma. The procedure involves placing a shunt in the eye to relieve intraocular pressure by creating a pathway for draining aqueous humor from the anterior chamber of the eye. The shunt is typically positioned in the eye such that it creates a drainage pathway between the anterior chamber of the eye and a region of lower pressure. Such fluid flow pathways allow for aqueous humor to exit the anterior chamber.

### SUMMARY

The importance of lowering intraocular pressure in delaying glaucomatous progression is well documented. When drug therapy fails, or is not tolerated, surgical intervention is warranted. There are various surgical filtration methods for lowering intraocular pressure by creating a fluid flow-path between the anterior chamber and the subconjunctival tissue. In one particular method, an intraocular shunt is implanted with an inserter by directing a needle, which holds the shunt through the cornea, across the anterior chamber, and through the trabecular meshwork and sclera, and into the subconjunctival space. See, for example, U.S. Patent No. 6,544,249, U.S. Patent Application Publication No. 2008/0108933, and U.S. Patent No. 6,007,511, the entireties of each of which are incorporated herein by reference.

Clinicians may implant the intraocular shunt using an ab externo procedure or an ab interno procedure, depending on the needs of the patient and the selected treatment. In an ab externo approach, the clinician may enter through the conjunctiva and inwards through the sclera, as described in the present applicant's publications, including U.S. Patent No. 10, 470,927, and U.S. Patent No. 10,463,537. In an ab interno approach, the clinician may enter through the cornea, across the anterior chamber, through the trabecular meshwork and sclera.

In accordance with an aspect of some embodiments disclosed herein is the realization that some clinicians may prefer to reposition a shunt relative to a bevel of the needle in preparation for surgery. For example, some clinicians may prefer to position the shunt in a certain location relative to a bevel of the needle when implanting the intraocular shunt using an ab interno procedure, but may prefer that the shunt be positioned at a different location relative to the bevel of the needle when performing an ab externo procedure. Moreover, certain anatomies, shunt types and/or materials, and/or procedures may require such repositioning, in order to enable the clinician to most advantageously deliver the shunt.

Further, in accordance with an aspect of some embodiments disclosed herein is the realization of the need for an intraocular shunt inserter that permits a clinician to quickly, reliably, and accurately reposition the shunt relative to the bevel of the needle according to their own preference, for performing different surgical techniques or approaches (e.g., ab externo procedure or ab interno), or in response to other shunt or patient factors and conditions.

Accordingly, the present disclosure contemplates these problems, provides solutions to these problems, and relates to the realization that precision can be increased while reducing operator effort, preparation time, cost, potential trauma to the patient, and surgery time, in some embodiments, implementing certain advantageous features in a shunt inserter.

Some embodiments disclosed herein provide an intraocular shunt inserter that includes a component driver including an actuation member, wherein the component driver elongates from a first length to a second length upon movement of the actuation member for changing a longitudinal relative position of a distal end portion of the plunger or a distal end portion of the needle. The first length of the component driver can position the shunt for ab externo treatment, and the second length of the component driver can position the shunt for ab interno treatment.

The component driver can be coupled to, part of, or control a movement or position of a plunger or needle of the inserter. Optionally, the inserter can include two component drivers, one component driver coupled to, part of, or control a movement or position of the plunger and another component driver coupled to, part of, or control a movement or position of the needle of the inserter.

Some embodiments disclosed herein provide an intraocular shunt inserter that includes a plunger driver coupled to a proximal end portion of the plunger, the plunger driver including an actuation member, wherein the plunger driver elongates from a first length to a second length upon movement of the actuation member for advancing the shunt relative to the bevel of the needle. The first length of the plunger driver can position the shunt for ab externo treatment, and the second length of the plunger driver can position the shunt for ab interno treatment.

Some embodiments disclosed herein provide an intraocular shunt inserter that includes a needle driver coupled to a proximal end portion of the needle, the needle driver including an actuation member, wherein the plunger driver foreshortens from a first length to a second length upon movement of the actuation member for retracting the bevel of the needle relative to the shunt. The first length of the needle driver can position the needle relative to the shunt for ab externo treatment, and the second length of the needle drive can position needle relative to the shunt for ab interno treatment.

Optionally, the actuation member can include an oblong cam, wherein the oblong cam is rotatable within a body of the plunger driver about an axis oriented perpendicular relative to a longitudinal axis of the inserter. The oblong cam can include a major axis and a minor axis, and the plunger driver elongates from the first length to the second length by rotating the oblong cam to align the major axis with the longitudinal axis of the inserter. The actuation member can positively displace the plunger driver from the first length to the second length.

Some embodiments disclosed herein provide an intraocular shunt inserter that includes a needle driver and a plunger driver, and an actuation mechanism to change the length of both the needle driver and the length of the plunger driver to adapt the inserter for either ab externo procedures or ab interno procedures. The plunger driver and the needle driver can move in opposing directions upon actuation.

Optionally, the inserter can include a removable key to releasably engage with the actuation member. The removable key can also prevent release of the shunt before intended.

An operator can operate the inserter by moving a plunger driver actuation member to change a plunger driver from a first plunger driver length to a second plunger driver length to advance the shunt relative to the bevel of the needle, The operator can also operate the inserter by moving a needle driver actuation member to change a needle driver from a first needle driver length to a second needle driver length to move the bevel of the needle relative to the shunt.

Optionally, the operator can rotate one or more cam mechanisms to extend the plunger driver and/or retract the needle driver, positioning the shunt relative to the bevel of the needle.

### BRIEF DESCRIPTION OF THE DRAWINGS

The accompanying drawings, which are included to provide further understanding of the subject technology and are incorporated in and constitute a part of this specification, illustrate aspects of the disclosure and together with the description serve to explain the principles of the subject technology.
Figure 1 is a schematic view of a procedure for implanting an intraocular shunt into an eye using an inserter, according to some embodiments.
Figure 2 is a perspective view of an inserter for implanting an intraocular shunt into an eye, according to some embodiments.
Figure 3 is a perspective, exploded view of the inserter shown in Figure 2, according to some embodiments.
Figure 4 is a perspective, exploded view of a drive assembly of the inserter shown in Figure 2, according to some embodiments.
Figure 5 is a perspective, exploded view of the needle assembly and the plunger assembly shown in Figure 4, according to some embodiments.
Figure 6 is a perspective, exploded view of the needle assembly and the plunger assembly shown in Figure 5 in an actuated position, according to some embodiments.
Figure 7 is a perspective view of the plunger assembly shown in Figure 4, according to some embodiments.
Figure 8 is an elevation view of the plunger driver shown in Figure 7, according to some embodiments.
Figure 9 is an elevation view of the plunger driver shown in Figure 8 in an actuated position, according to some embodiments.
Figure 10 is a perspective view of the needle assembly shown in Figure 4, according to some embodiments.
Figure 11 is an elevation view of the needle driver shown in Figure 10, according to some embodiments.
Figure 12 is an elevation view of the needle driver shown in Figure 11 in an actuated position, according to some embodiments.
Figure 13 is an elevation view of a rotatable key, according to some embodiments.

### DETAILED DESCRIPTION

In the following detailed description, numerous specific details are set forth to provide a full understanding of the subject technology. It should be understood that the subject technology may be practiced without some of these specific details. In other instances, well-known structures and techniques have not been shown in detail so as not to obscure the subject technology.

Glaucoma is a disease in which the optic nerve is damaged, leading to progressive, irreversible loss of vision. It is typically associated with increased pressure of the fluid (i.e., aqueous humor) in the eye. Untreated glaucoma leads to permanent damage of the optic nerve and resultant visual field loss, which can progress to blindness. Once lost, this damaged visual field cannot be recovered.

In conditions of glaucoma, the pressure of the aqueous humor in the eye (anterior chamber) increases and this resultant increase of pressure can cause damage to the vascular system at the back of the eye and especially to the optic nerve. The treatment of glaucoma and other diseases that lead to elevated pressure in the anterior chamber involves relieving pressure within the anterior chamber to a normal level.

Glaucoma filtration surgery is a surgical procedure typically used to treat glaucoma. The procedure involves placing a shunt in the eye to relieve intraocular pressure by creating a pathway for draining aqueous humor from the anterior chamber of the eye. The shunt is typically positioned in the eye such that it creates a drainage pathway between the anterior chamber of the eye and a region of lower pressure. Various structures and/or regions of the eye having lower pressure that have been targeted for aqueous humor drainage include Schlemm's canal, the subconjunctival space, the episcleral vein, the suprachoroidal space, the intra-Tenon's adhesion space, and the subarachnoid space. Shunts may be implanted using an ab externo approach (e.g., entering through the conjunctiva and inwards through the sclera) or an ab interno approach (e.g., entering through the cornea, across the anterior chamber, through the trabecular meshwork and sclera). For example, ab externo approaches for implanting an intraocular shunt in the subconjunctival space are shown for example in Horvath et al. (U.S. Patent No 10,470,927) and Horvath et al. (U.S. Patent Application Publication No. 2017/0348150), the contents of each which are incorporated by reference herein in its entirety. For example, ab interno approaches for implanting an intraocular shunt in the subconjunctival space are shown for example in Yu et al. (U.S. Patent No. 6,544,249 and U.S. Patent Application Publication No. 2008/0108933) and Prywes (U.S. Patent No. 6,007,511), the contents of each of which are incorporated by reference herein in its entirety.

Some methods can involve inserting into the eye a hollow shaft configured to hold an intraocular shunt. In some embodiments, the hollow shaft can be a component of a deployment device that may deploy the intraocular shunt. The hollow shaft can be coupled to a deployment device or be part of the deployment device itself. The deployment devices can include devices such as those as described in co-owned U.S. Patent No. 9,585,790, U.S. Patent No. 8,721,792, U.S. Patent No. 8,852,136, and U.S. Patent Application Publication No. 2012/0123434, filed on November 15, 2010, the contents of each of which are incorporated by reference herein in their entireties.

In accordance with some embodiments, the inserter can be advanced into the eye via an ab-interno or an ab-externo approach. Thereafter, the shunt can be deployed from the shaft into the eye such that the shunt forms a passage from the anterior chamber into an area of lower pressure, such as Schlemm's canal, the subconjunctival space, the episcleral vein, the suprachoroidal space, the intra-Tenon's adhesion space, the subarachnoid space, or other areas of the eye. The hollow shaft is then withdrawn from the eye. Methods for delivering and implanting bioabsorbable or permanent tubes or shunts, as well as implantation devices for performing such methods, are generally disclosed in applicant's applications, including U.S. Patent Application Publication Nos. 2012/0197175, 2015/0011926, and 2016/0354244, U.S. Patent Application No. 15/613,018, as well as in U.S. Pat. Nos. 6,007,511, 6,544,249, 8,852,136, and 9,585,790 each of which are incorporated by reference in their entireties.

Some methods can be conducted by making an incision in the eye prior to insertion of the deployment device. However, in some instances, the method may be conducted without making an incision in the eye prior to insertion of the deployment device. In some embodiments, the shaft that is connected to the deployment device has a sharpened point or tip. In some embodiments, the hollow shaft is a needle. Exemplary needles that may be used are commercially available from Terumo Medical Corp. (Elkington, Md). In some embodiments, the needle can have a hollow interior and a beveled tip, and the intraocular shunt can be held within the hollow interior of the needle. In some embodiments, the needle can have a hollow interior and a triple ground point or tip.

Some methods can be conducted without needing to remove an anatomical portion or feature of the eye, including but not limited to the trabecular meshwork, the iris, the cornea, or aqueous humor. Some methods can be conducted without inducing substantial ocular inflammation, such as subconjunctival blebbing or endophthalmitis. Some methods can be achieved using an ab interno approach by inserting the hollow shaft configured to hold or carry the intraocular shunt as it is advanced through the cornea, across the anterior chamber, through the trabecular meshwork, and into the intra-scleral or intra-Tenon's adhesion space. However, some methods may be conducted using an ab externo approach.

In some methods conducted using an ab interno approach, the angle of entry through the cornea can be altered to affect optimal placement of the shunt in the intra-Tenon's adhesion space. The hollow shaft can be inserted into the eye at an angle above or below the corneal limbus, in contrast with entering through the corneal limbus.

For example, the hollow shaft can be inserted from about 0.25 mm to about 3.0 mm above the corneal limbus. The shaft can be inserted from about 0.5 mm to about 2.5 mm above the corneal limbus. The shaft can also be inserted from about 1.0 mm to about 2.0 mm above the corneal limbus, or any specific value within any of these ranges. For example, the hollow shaft can be inserted above the corneal limbus at distances of about: 1.0 mm, 1.1 mm, 1.2 mm, 1.3 mm, 1.4 mm, 1.5 mm, 1.6 mm, 1.7 mm, 1.8 mm, 1.9 mm, or 2.0 mm.

An ab interno approach or procedure may require or benefit from a shunt that is positioned differently relative to the bevel of the needle compared to the position of the shunt for ab externo procedures. For example, for an ab externo procedure it may be beneficial if the distal end of the shunt is positioned further proximal to the bevel of the needle than for an ab interno procedure. As noted above, existing inserters may not be able to, or may not easily allow for a clinician to reposition the shunt relative to the bevel of the needle prepare for an ab externo and/or ab interno procedure. The present disclosure provides various embodiments of methods and devices that can enable an operator easily position the shunt relative to the bevel of the needle to configure the inserter for either an ab externo procedure or an ab interno procedure.

Further, in some embodiments, placement of the shunt farther from the limbus at the exit site, as provided by an angle of entry above the limbus, can provide access to more lymphatic channels for drainage of aqueous humor, such as the episcleral lymphatic network, in addition to the conjunctival lymphatic system. A higher angle of entry also results in flatter placement in the intra-Tenon's adhesion space so that there is less bending of the shunt.

As discussed in U.S. Patent No. 8,852,136, the entirety of which is incorporated herein by reference, in some embodiments, to ensure proper positioning and functioning of the intraocular shunt, the depth of penetration into the intra-Tenon's adhesion space may be important when performing some methods.

In some methods, the distal tip of the hollow shaft can pierce the sclera and intra-Tenon's adhesion space without coring, removing or causing major tissue distortion of the surrounding eye tissue. The shunt is then deployed from the shaft. Preferably, a distal portion of the hollow shaft (as opposed to the distal tip) completely enters the intra-Tenon's adhesion space before the shunt is deployed from the hollow shaft.

In accordance with some embodiments, the hollow shaft can comprise a flat bevel needle, such as a needle having a triple-ground point. The tip bevel can first pierce through the sclera and into the intra-Tenon's adhesion space by making a horizontal slit. In some methods, the needle can be advanced even further such that the entire flat bevel penetrates into the intra-Tenon's adhesion space, to spread and open the tissue to a full circular diameter.

Further, in accordance with an aspect of some methods, the intra-Tenon's channel can be urged open by the flat bevel portion of the needle so that the material around the opening is sufficiently stretched and a pinching of the shunt in that zone is avoided, thus preventing the shunt from failing due to the pinching or constriction. Full entry of the flat bevel into the intra-Tenon's adhesion space causes minor distortion and trauma to the local area. However, this area ultimately surrounds and conforms to the shunt once the shunt is deployed in the eye.

In some embodiments, the inserter can function as a one-handed device in order to allow an operator to keep their other hand on a fixation device that holds the eye, such as a hook. This can improve surgical control and placement accuracy and makes the surgery easier as well.

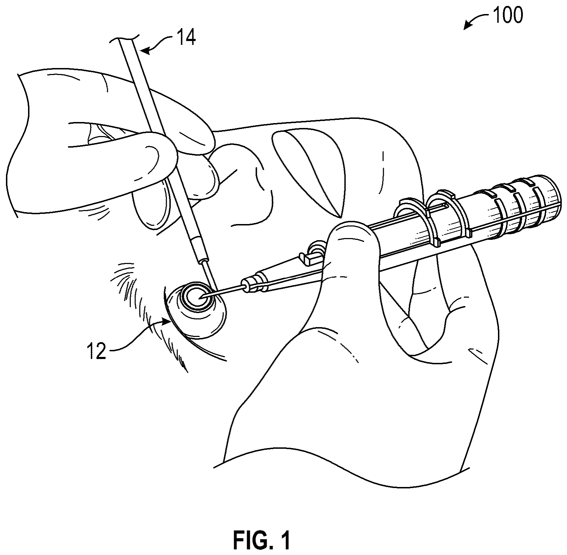

An illustration of a procedure for treating an eye 12 is shown in Figure 1. Figure 1 illustrates the use of a hook 14 for holding the eye 12 and an inserter 100 for introducing an intraocular shunt into the eye.

Embodiments of an intraocular shunt inserter are described herein. The shunt inserter can include a needle defining a lumen, A plunger can be partially disposed within the lumen of the needle. The plunger can advance a shunt through the needle to introduce the intraocular shunt into the eye.

A component driver can include an actuation member to move the plunger and/or the needle to prepare the inserter for ab externo or ab interno procedures. In some embodiments, the component driver can be actuated to change the position of the shunt relative to the bevel of the needle. The component driver can be coupled to, part of, or control a movement or position of a plunger or needle of the inserter.

For example, optionally, the component driver can be elongated or foreshortened from a first length to a second length to change the longitudinal relative position of the distal end portion of the plunger.

Further optionally, the component driver can be elongated or foreshortened from a first length to a second length to change the longitudinal relative position of the distal end portion of the needle.

Optionally, the inserter can include two component drivers, one component driver coupled to, part of, or control a movement or position of the plunger and another component driver coupled to, part of, or control a movement or position of the needle of the inserter.

In some embodiments, the component driver can be coupled to the needle to change the relative position of the distal end portion of the needle. In some embodiments, the component driver can be coupled to the plunger to change the relative position of the distal end portion of the plunger.

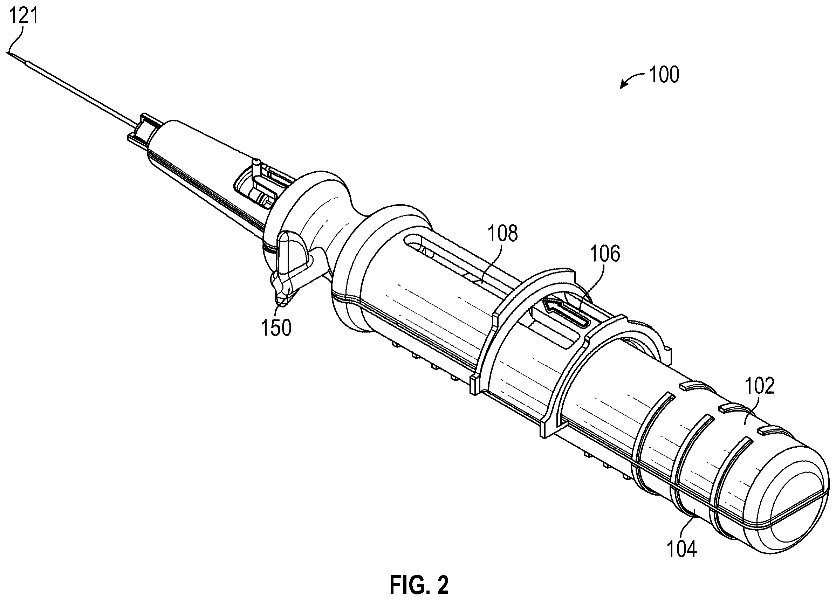

Figures 2-13 illustrate further details of the inserter 100 shown in Figure 1. The inserter 100 can be actuated using a single hand, thus facilitating use of the inserter by an operator. The inserter 100 can comprise an upper housing 102, a lower housing 104, a slider component 106, and a needle 121. As shown in Figure 2, the inserter 100 can be configured such that the slider component 106 is slidable along an elongate slot 108 of the housing 102. The slider component 106 can be selectively movable by an operator in order to actuate movement of components of the inserter 100.

For example, when the slider component 106 moves distally along the slot 108 (i.e., in a direction toward the needle 121), the slider component 106 can result in or cause a shunt (not shown) to be advanced within the needle 121, and in some embodiments, released from the needle 121. In accordance with some embodiments discussed further herein, movement of the slider component 106 can result in translational and/or rotational movement of components of the inserter 100. The sliding movement of the slider component 106 can be converted into rotational movement, which can thereafter be converted to movement along a longitudinal axis of the inserter 100. One of the benefits of this innovative and complex movement-conversion mechanism is that it enables embodiments of the inserter to provide precise, measured movements of its components within a compact assembly.

In the depicted example, the inserter 100 can be configured for ab externo procedures or ab interno procedures by rotating the removable key 150 prior to an implantation procedure. As described herein, the removable key 150 can be positioned in a vertical position (as shown in FIG. 2) to position the shunt relative to the needle 121 for ab externo procedures. Similarly, the removable key 150 can be rotated or otherwise positioned in a horizontal position to move the shunt relative to the needle 121 for ab interno procedures.

After configuring the inserter 100 for the ab externo procedure or the ab interno procedure, the removable key 150 can be removed or otherwise separated from the inserter 100. In some embodiments, the removable key 150 functions as an interlock to prevent the actuation of the inserter 100 until the removable key 150 is removed. Optionally, the removable key 150 can prevent the slider 106 from advancing until the removable key 150 is removed from the inserter 100.

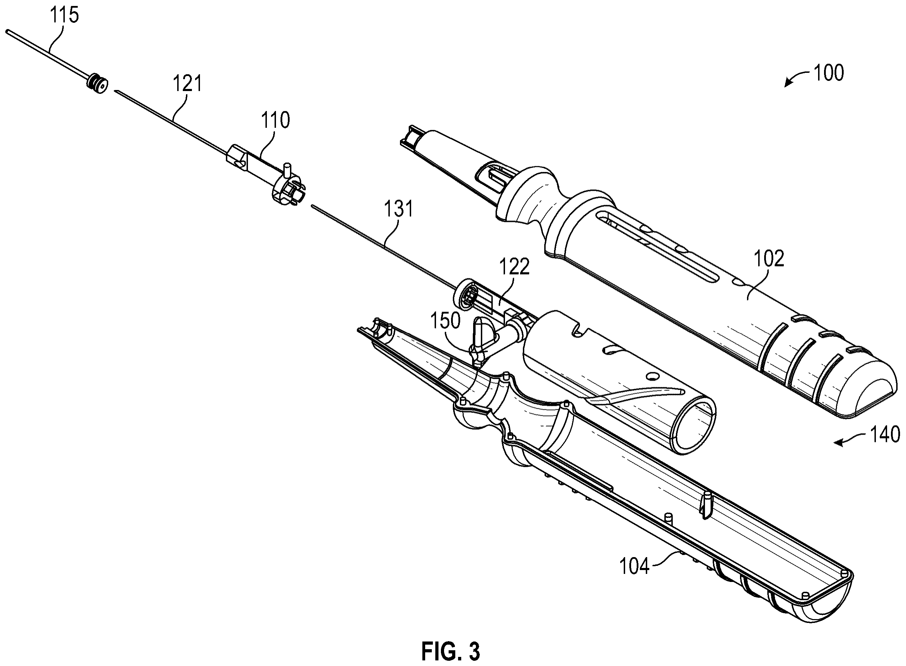

As illustrated in Figure 3, a plunger 131 can be disposed within the needle 121 to advance the shunt for implantation. The needle 121 can comprise a 25 GA or 27 GA needle. The plunger 131 can be slidably movable within a lumen of the needle 121 along a longitudinal axis of the inserter 100. Further, the needle 121 can be slidably movable within a lumen of the sleeve component 115 along the longitudinal axis. In some embodiments, the needle 121 can be attached to a movable needle base 110.

Each of the needle 121 and the plunger 131 can be coupled to respective drive components of a drive assembly 140 disposed within the housing 102 and 104. When in the assembled state, the inserter 100 can be configured such that the needle 121, the plunger 131, and the sleeve component 115 are aligned along or coaxial with the longitudinal axis. Some drive assemblies for actuating a plunger and for withdrawing a needle of an inserter are disclosed in U.S. Patent Application Nos. 13/336,803, 12/946,645, 12/620,564, 12/946,653, 12/946,565, and 11/771,805 and U.S. Patent No. 9,585,790, the entireties of which are incorporated herein by reference.

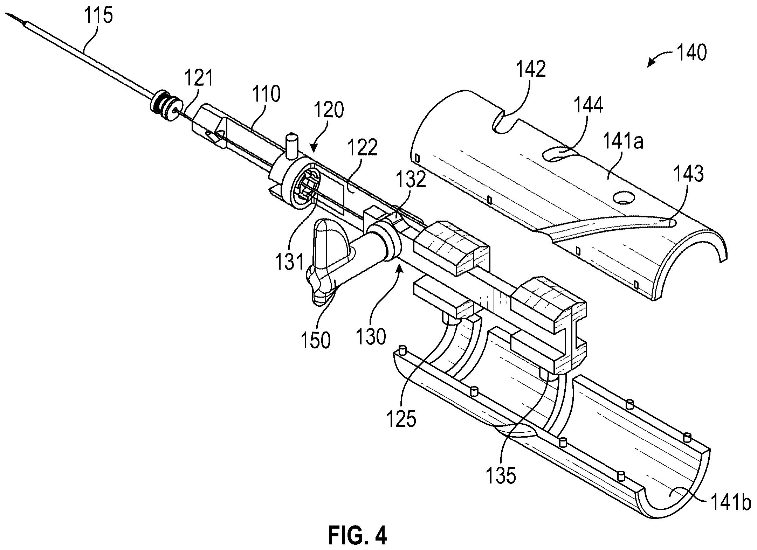

Referring to Figures 3 and 4, the needle 121, the plunger 131, and the sleeve 115 can be operably coupled to the drive assembly 140 and/or the housing 102. For example, a needle assembly 120 can operably couple the needle 121 to the drive assembly 140. The needle assembly 120 can include the needle 121, a needle base 110 and a needle driver 122. In the depicted example, the needle 121 can be coupled to the needle base 110. The needle base 110 can be fixedly coupled to a proximal end portion of the needle 121 such that rotational and longitudinal movement between the needle 121 and the needle base 110 is restricted or prevented. The needle base 110 can be enclosed within a distal end portion of the housing 102 when the inserter 100 is assembled. Further, as illustrated in Figure 4 and discussed further below, the needle base 110 can be coupled to the drive assembly 140 via the needle driver 122.

Further, as shown in Figure 4, a plunger assembly 130 can operably couple the plunger 131 to the drive assembly 140. The plunger assembly 130 can include the plunger 131 and a plunger driver 132. The plunger driver 132, can be fixedly coupled to a proximal end portion or midsection of the plunger 131 to restrict or prevent rotational and longitudinal movement of the plunger 131 relative to the plunger driver 132. Further, as illustrated in Figure 4 and discussed further below, the plunger driver 132 can be coupled to the drive assembly 140.

Furthermore, the sleeve component 115 can be coupled to a portion of the housing 102 and 104. The sleeve 115 can be coupled so as to prevent rotational and longitudinal movement between the sleeve component 115 and the housing 102 and 104.

As noted above, the needle 121 and the plunger 131 can be operably coupled to the drive assembly 140. Such coupling can occur via the needle driver 122 and the plunger driver 132. In turn, the needle driver 122 and the plunger driver 132 can be coupled to one or more drive components that engage with the drive assembly 140 to the housing 102.

In accordance with some embodiments, the drive assembly 140 can be coupled to the needle 121 and the plunger 131 to actuate movement along the longitudinal axis of the needle 121 and the plunger 131 relative to the housing 102 and 104. For example, the drive assembly 140 can be configured to rotate or slide within the housing 102. The drive assembly 140 can transfer a longitudinal or axial force along the longitudinal axis to the needle component 121 and/or the plunger 131, independently or at the same time, to result in movement of the needle 121 and the plunger 131 relative to the housing 102 and 104 along the longitudinal axis.

As discussed herein, motion of the slider component 106 can result in motion of the drive assembly 140 and thereby result in motion of components of the drive assembly 140 relative to the housing 102 and 104. Some embodiments can be configured such that the slider component 106 can be longitudinally movable or slidable along the longitudinal axis relative to the housing 102 and 104 in order to drive or result in linear motion of the needle 121 and the plunger 131 and consequently a shunt.

As shown in Figure 4, the drive assembly 140 can comprise drive components 141a and 141b configured to engage with the needle driver 122 and plunger driver 132. In some embodiments, longitudinal or linear motion of the slider component 106 along the longitudinal axis can be converted to result in rotation of the drive components 141a and 141b of the drive assembly 140, which can then be converted to result in longitudinal or linear motion of the needle 121 and the plunger 131 along the longitudinal axis relative to the housing 102 and 104. In accordance with some embodiments, motion of the components along the longitudinal axis can be parallel relative to the longitudinal axis.

Figure 4 also illustrates an embodiment of the drive components 141a and 141b. The drive components 141a and 141b can comprise a groove 143 that can be configured to engage with a corresponding protrusion (not shown) of the slider component 106. Further, the drive components 141a and 141b can also comprise first and second driving grooves 142, 144 that can be configured to slidingly engage corresponding protrusions of the needle driver 122 and the plunger driver 132. Thus, the slider component 106 can comprise a protrusion, the needle driver 122 can comprise a protrusion 125, and the plunger driver 132 can comprise a protrusion 135. This arrangement of slots and protrusions can facilitate the transfer of motion from the slider component 106 to the respective ones of the needle 121 and the plunger 131. Further, the plunger driver 132 and the needle driver 122 can comprise rounded bodies that contact and slide against an inner guide surface of the drive components 141a and 141b when seated within the drive components 141a and 141b.

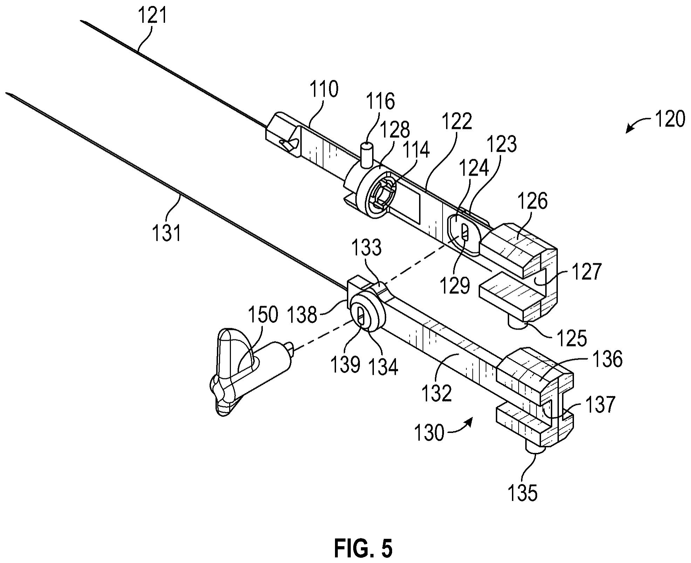

As described herein, clinicians may select an ab externo approach or an ab interno approach to implant the shunt using the inserter 100 depending on the patient's needs and other factors. With reference to Figure 5, the inserter 100 can be configured to position the shunt within the needle 121 for an ab externo approach or for an ab interno approach. As illustrated, the needle assembly 120 and/or the plunger assembly 130 can be configured to adjust the position of the shunt to be suitable for an ab externo approach or for an ab interno approach prior to the implantation procedure.

In some embodiments, portions of the needle assembly 120 and/or the plunger assembly 130 can be elongated, foreshortened, or otherwise changed to adjust the relative position of the needle 121 and/or the plunger 131 and consequently the relative position of the shunt for either ab externo or ab interno approaches. In the depicted example, the needle driver 122 and/or the plunger driver 132 can be elongated, foreshortened, or otherwise changed to adjust the position of the shunt.

For example, as shown in Figure 5 the plunger driver 132 can be positioned in a foreshortened position and the needle driver 122 can be positioned in an elongated position for an ab externo approach. In some embodiments, the plunger driver 132 can be foreshortened by about 0.25 mm relative to an elongated configuration. Optionally, the plunger driver 132 can be foreshortened by about 0.05 mm, about 0.10 mm, about 0.15 mm, about 0.20 mm, about 0.30 mm, about 0.40 mm, or about 0.50 mm. In some embodiments, the needle driver 122 can be elongated approximately 1 millimeter relative to a foreshortened configuration. Optionally, the needle driver 122 can be elongated approximately about 0.8 mm, about 0.9 mm, about 1.1 mm, about 1.2 mm, about 1.3 mm, or about 1.5 mm. By foreshortening the plunger driver 132 and elongating the needle driver 122, the shunt can repositioned proximally relative to the bevel of the needle 121. In the ab externo configuration, the tip of the plunger 131 and therefore the shunt can be positioned approximately 7 mm from the bevel of the needle 121. Optionally, the tip of the plunger 131 can be positioned by about 4 mm, about 5 mm, about 6 mm, about 8 mm, about 9 mm, about 10 mm, or about 11 mm from the bevel of the needle 121 prior to actuation of the inserter 100. Upon actuation of the inserter 100, the plunger 131 can be advanced about 6 mm relative to the needle 121. In some embodiments, upon actuation of the inserter 100, the plunger 131 can be advanced by about 4 mm, about 5 mm, about 6 mm, about 8 mm, about 9 mm, about 10 mm, or about 11 mm relative to the needle 121.

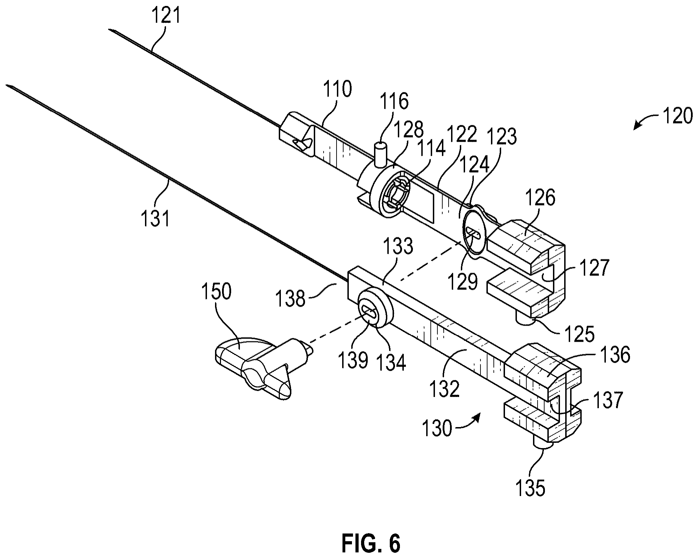

As shown in Figure 6, the removable key 150 can be actuated to move the plunger driver 132 to an elongated position and the needle driver 122 to a foreshortened position for an ab interno approach. In some embodiments, the plunger driver 132 can be elongated by about 0.25 mm relative to an foreshortened configuration. Optionally, the plunger driver 132 can be elongated by about 0.05 mm, about 0.10 mm, about 0.15 mm, about 0.20 mm, about 0.30 mm, about 0.40 mm, or about 0.50 mm. In some embodiments, the needle driver 122 can be foreshortened approximately about 1 millimeter relative to an elongated configuration. Optionally, the needle driver 122 can be foreshortened by about 0.8 mm, about 0.9 mm, about 1.0 mm, about 1.1 mm, about 1.2 mm, about 1.3 mm, or about 1.5 mm. By elongating the plunger driver 132 and foreshortening the needle driver 122, the shunt can be positioned distally relative to the ab externo shunt position. In the ab interno configuration, the plunger 131 can extend and the needle 121 can retract to advance the shunt by about 1.25 mm relative to the needle 121 compared to the ab externo configuration. Therefore, in the ab interno configuration, the tip of the plunger 131 and therefore the shunt can be positioned about 5.75 mm from the bevel of the needle 121. Optionally, the tip of the plunger 131 can be positioned about 3 mm, about 4 mm, about 5 mm, about 5.5 mm, about 6 mm, about 7 mm, or about 8 mm from the bevel of the needle 121 prior to actuation of the inserter 100. Upon actuation of the inserter 100, the plunger 131 can be advanced approximately 6 mm relative to the needle 121. In some embodiments, upon actuation of the inserter 100, the plunger 131 can be advanced by about 4 mm, about 5 mm, about 6 mm, about 8 mm, about 9 mm, about 10 mm, or about 11 mm relative to the needle 121.

As can be appreciated, the inserter 100 can be provided with a default configuration that is suitable for ab externo or ab interno procedures. The removable key 150 can be used to change the configuration from an ab externo configuration to an ab interno configuration or vice versa.

Optionally, the removable key 150 can extend through portions of the needle driver 122 and/or the plunger driver 132 to prevent movement of the needle assembly 120 and/or the plunger assembly 130 relative to the housing 102 and 104, preventing inadvertent release of the shunt. Therefore, a clinician can utilize the removable key 150 to select the configuration of the inserter 100 and further prevent the release of the shunt until desired.

In some embodiments, the configuration of the inserter 100 can be changed by only altering the length of the needle assembly 120, only altering the length of the plunger assembly 130, or altering both the length of the needle assembly 120 and the length of the plunger assembly 130. In some embodiments the length of the needle driver 122 and the plunger driver 132 can be changed in opposing or different directions to change the configuration of the inserter 100.

Optionally, the length of the needle driver 122 and the plunger driver 132 can be changed simultaneously in a single action. In some embodiments, the nested arrangement of the needle driver 122 and the plunger driver 132 can allow the removable key 150 to engage with and actuate both the needle driver 122 and plunger driver 132 with a single action, simultaneously adjusting the length or configurations of the needle driver 122 and the plunger driver 132.

As can be appreciated, the needle driver 122 and/or the plunger driver 132 can utilize any suitable mechanism to elongate or foreshorten. As illustrated in Figures 5-11, the needle driver 122 and the plunger driver 132 can utilize cam mechanisms to adjust the length of the needle assembly 120 and/or the plunger assembly 130.

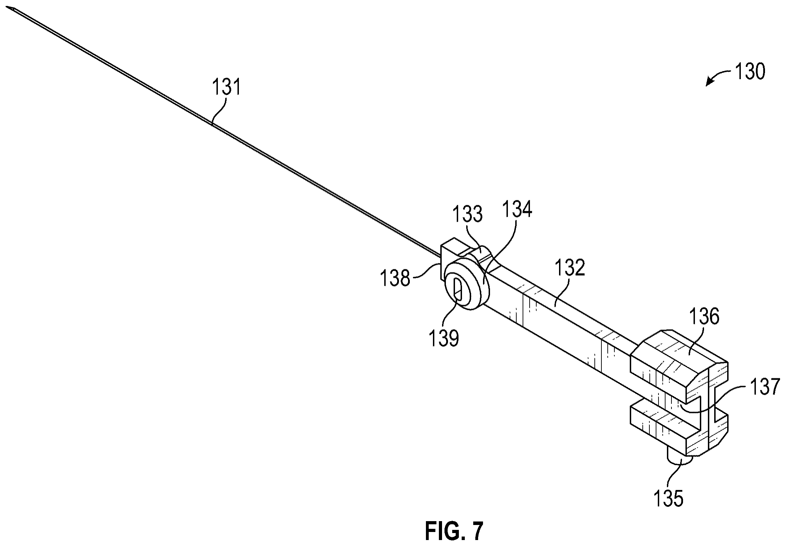

For example, the plunger assembly 130 allows for force or movement from the drive assembly 140 to actuate the plunger 131 and therefore the shunt. As shown in Figure 7, the proximal end of the plunger 131 can be affixed or otherwise coupled to the distal end 138 of the plunger driver 132. As described herein, the drive assembly 140 can engage with and actuate the plunger driver 132 via the protrusion 135. The protrusion 135 can extend from the proximal end 137 of the plunger driver 132. Optionally, the rounded portion 136 can allow the plunger driver 132 to slide freely and be aligned within the drive assembly 140.

In the depicted example, a cam mechanism 134 can elongate or foreshorten the plunger driver 132. In some embodiments, the cam mechanism 134 can move the distal end 138 of the plunger driver 132 further or closer to the proximal end 137. In some embodiments, the cam mechanism 134 comprises a rotatable generally oblong shape with a major axis A and a minor axis B, such that the major axis A is longer than the minor axis B. As illustrated, the cam mechanism 134 can be rotated by the keyed slot 139. The removable key 150 can engage with the keyed slot 139 to actuate the cam mechanism 134. Optionally, the keyed slot 139 can be aligned with the major axis A of the cam mechanism 134.

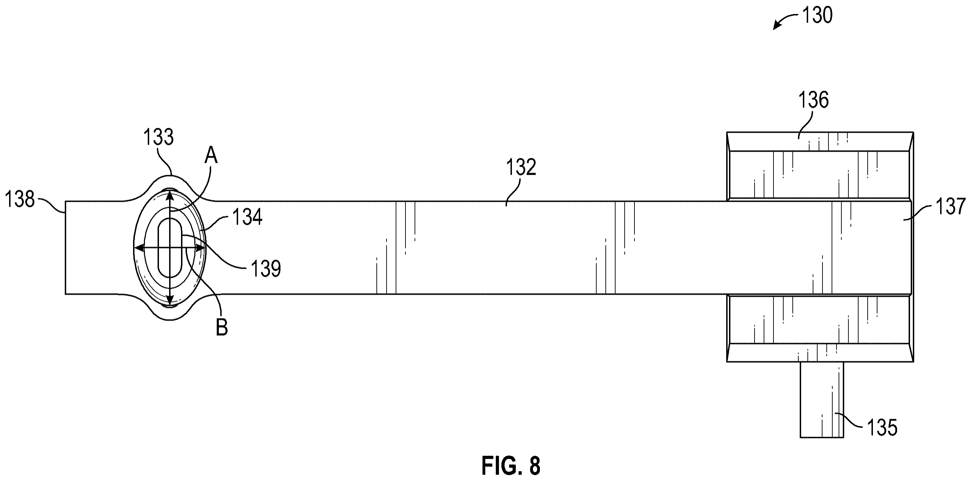

As illustrated, the cam mechanism 134 can be disposed within an aperture 133. The aperture 133 can extend at least partially through the plunger driver 132. As described herein, the cam mechanism 134 can be actuated to positively displace the aperture 133, extending the plunger driver 132 or foreshortening the plunger driver 132. As illustrated in Figure 8, the cam mechanism 134 can be rotated within the aperture 133 to align the major axis A of the cam mechanism 134 in a direction generally perpendicular to the length of the plunger driver 132, displacing the walls of the aperture 133 (e.g., creating a bulged portion) and foreshortening the plunger driver 132. In some embodiments, the foreshortened position of the plunger driver 132 corresponds with an ab externo treatment.

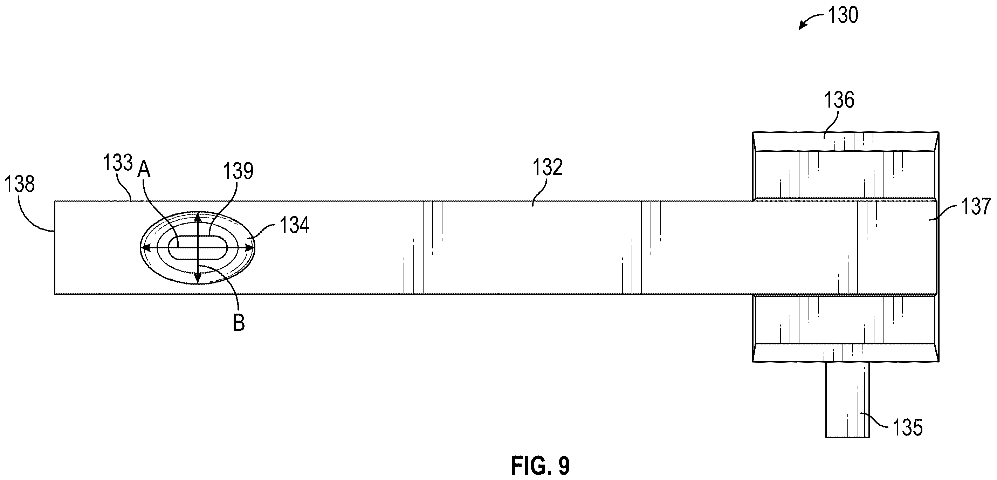

As illustrated in Figure 9, the cam mechanism 134 can be rotated within the aperture 133 to align the major axis A of the cam mechanism 134 in a direction generally parallel to the length of the plunger driver 132, displacing the walls of the aperture 133 and elongating the plunger driver 132. In some embodiments, the elongated position of the plunger driver 132 corresponds with an ab interno treatment. In some embodiments, the rotation or actuation of the cam mechanism 134 does not elastically deform the plunger driver 132. In other words, the plunger driver 132 is may not be biased to return to any particular length without actuation.

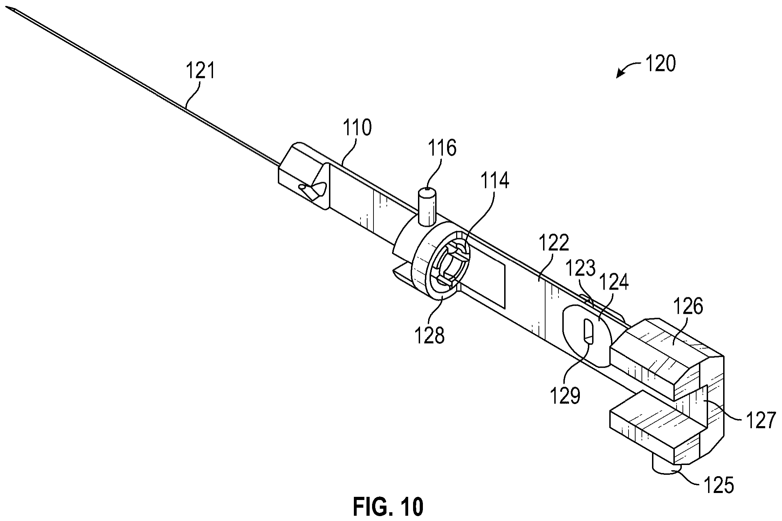

Similarly, the needle assembly 120 allows for force or movement from the drive assembly 140 to actuate the needle 121. As shown in Figure 10, the proximal end of the needle 121 can be affixed or otherwise coupled to the distal end 128 of the needle driver 122. Optionally, the proximal end of the needle 121 can be affixed to a needle base 110. The needle base 110 can be engaged with the distal end of the needle driver 122. The proximal end 114 of the needle base 110 can have a "snap" or friction fit with the distal end 128 of the needle driver 122. The needle driver 122 can have a complimentary or receiving aperture at the distal end 128. Optionally, the needle base 110 can include a rotational adjustment feature 116 to allow the clinician to adjust the rotation of the bevel of the needle 121.

As described herein, the drive assembly 140 can engage with and actuate the needle driver 122 via the protrusion 125. The protrusion 125 can extend from the proximal end 127 of the needle driver 122. Optionally, the rounded portion 126 can allow the needle driver 122 to slide freely and be aligned within the drive assembly 140.

In the depicted example, a cam mechanism 124 can elongate or foreshorten the needle driver 122 to adjust the position of the needle bevel relative to the distal end of the plunger 131, and therefore shunt. In some embodiments, the cam mechanism 124 can move the distal end 128 of the needle driver 122 further or closer to the proximal end 127. In some embodiments, the cam mechanism 124 comprises a rotatable generally oblong shape with a major axis C and a minor axis D, such that the major axis C is longer than the minor axis D. As illustrated, the cam mechanism 124 can be rotated by the keyed slot 129. The removable key 150 can engage with the keyed slot 129 to actuate the cam mechanism 124. In some embodiments, the keyed slot 129 can be aligned with the major axis C of the cam mechanism 124 (not pictured).

In some embodiments the keyed slot 129 of the needle assembly 120 can be aligned with the keyed slot 139 of the plunger assembly 130, allowing the removable key 150 to engage with both the needle assembly 120 and the plunger assembly 130. Advantageously, the plunger assembly 130 and the needle assembly 120 can be configured simultaneously with a single action or rotation of the removable key 150.

As illustrated in Figure 5, the needle driver 122 can comprise a body having an aperture 123 extending therethrough. The cam mechanism 124 can be disposed through and rotatable within the aperture 123. As described herein, the cam mechanism 124 can be actuated to positively displace the aperture 123, extending the needle driver 122 or foreshortening the needle driver 122.

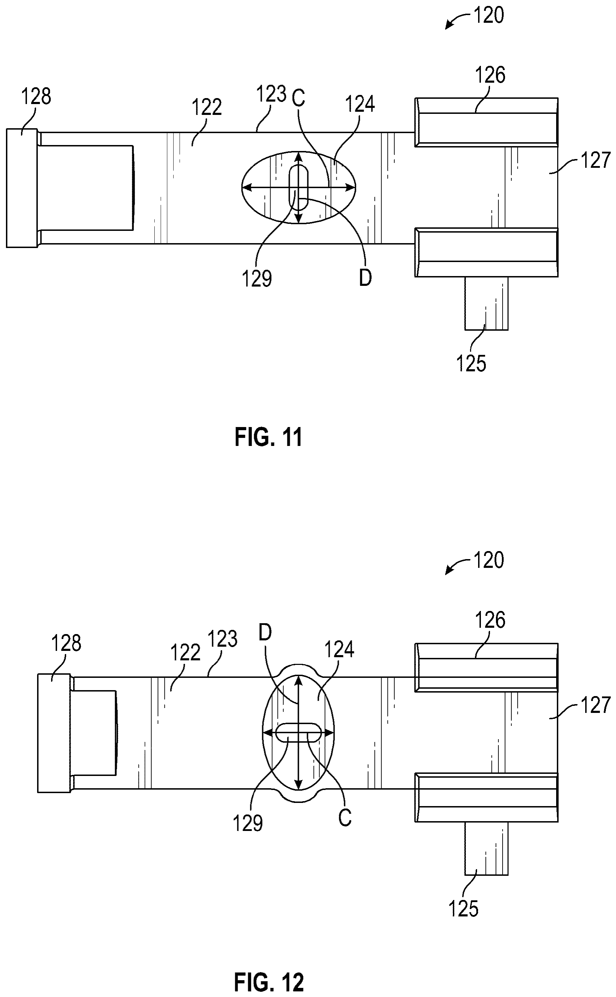

In some embodiments, as illustrated in Figure 11, the cam mechanism 124 can be rotated within the aperture 123 to align the major axis C of the cam mechanism 124 in a direction generally parallel to the length of the needle driver 122, displacing the walls of the aperture 123 and elongating the needle driver 122. In some embodiments, the elongated position of the needle driver 122 corresponds with an ab externo treatment.

As illustrated in Figure 12, the cam mechanism 124 can be rotated within the aperture 123 to align the major axis C of the cam mechanism 124 in a direction generally perpendicular to the length of the needle driver 122, displacing the walls of the aperture (e.g., creating a bulged portion), thereby foreshortening the needle driver 122. In some embodiments, the foreshortened position of the needle driver 122 corresponds with an ab interno treatment.

In some embodiments, the rotation or actuation of the cam mechanism 124 does not elastically deform the needle driver 122. In other words, the needle driver 122 is may not be biased to return to any particular length without actuation.

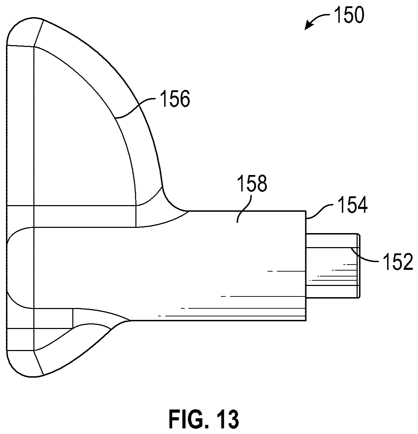

As illustrated in Figure 13, the removable key 150 can be used to actuate the needle assembly 120 and/or the plunger assembly 130. In some embodiments, the removable key 150 is provided with the inserter 100. The removable key 150 includes an extension portion 152 that is configured to engage with the slot 129 and/or slot 139. The extension portion 152 can extend through both slots 129 and 139 to actuate both the needle assembly 120 and the plunger assembly 130 simultaneously. The body 158 of the removable key 150 can abut the housing 102 and 104. In some embodiments, the surface 154 can contact the housing 102 and 104. Optionally, a wing 156 can extend from the body 158 of the removable key 150, allowing a clinician to easily rotate the removable key 150.

As described herein, in addition to configuring the inserter 100 for ab externo and ab interno procedures, the removable key 150 can engage with the slots 129 and 139 to prevent the inadvertent actuation of the inserter 100. Optionally, the removable key 150 can engage with the needle assembly 120 and/or the plunger assembly 130 to prevent the drive assembly 140 from actuating the needle assembly 120 and/or the plunger assembly 130 relative to the housing 102 and 104. Upon removing the removable key 150 from the housing 102 and 104, the drive assembly 140 can actuate the needle assembly 120 and/or the plunger assembly 130.

Although the detailed description contains many specifics, these should not be construed as limiting the scope of the subject technology but merely as illustrating different examples and aspects of the subject technology. It should be appreciated that the scope of the subject technology includes other embodiments not discussed in detail above. Various other modifications, changes and variations may be made in the arrangement, operation and details of the method and apparatus of the subject technology disclosed herein without departing from the scope of the present disclosure. Unless otherwise expressed, reference to an element in the singular is not intended to mean "one and only one" unless explicitly stated, but rather is meant to mean "one or more." In addition, it is not necessary for a device or method to address every problem that is solvable by different embodiments of the disclosure in order to be encompassed within the scope of the disclosure.

### Illustration of Subject Technology as Clauses

Various examples of aspects of the disclosure are described below as clauses for convenience. These are provided as examples, and do not limit the subject technology. The following numbered clauses also form part of the present disclosure.

Clause 1. An intraocular shunt inserter for treating glaucoma, comprising: a needle defining a lumen; and a plunger assembly comprising: a plunger at least partially disposed within the lumen of the needle, wherein the plunger is configured to advance a shunt through the lumen of the needle upon deployment; and a plunger driver coupled to a proximal end portion of the plunger, the plunger driver comprising an actuation member, wherein the plunger driver elongates from a first length to a second length upon movement of the actuation member for advancing a distal end portion of the plunger from a first position to a second position relative to the lumen.

Clause 2. The inserter of Clause 1, wherein the actuation member comprises an oblong cam, wherein the oblong cam is rotatable within the plunger driver about an axis oriented perpendicular relative to a longitudinal axis of the inserter.

Clause 3. The inserter of Clause 2, wherein the oblong cam comprises a major axis and a minor axis, and the plunger driver elongates from the first length to the second length by rotating the oblong cam to align the major axis with the longitudinal axis of the inserter.

Clause 4. The inserter of any one of the preceding Clauses, wherein the actuation member positively displaces the plunger driver from the first length to the second length.

Clause 5. The inserter of any one of the preceding Clauses, further comprising a removable key, wherein the removable key is releasably engaged with the actuation member to move the actuation member.

Clause 6. The inserter of Clause 5, wherein the removable key is releasably engageable with the actuation member.

Clause 7. The inserter of any one of the preceding Clauses, wherein the first position of the plunger corresponds to an ab externo treatment.

Clause 8. The inserter of any one of the preceding Clauses, wherein the second position of the plunger corresponds to an ab interno treatment.

Clause 9. An intraocular shunt inserter for treating glaucoma, comprising: a housing; a needle assembly comprising: a needle extending at least partially beyond a distal portion of the housing, the needle defining a lumen; and a needle driver coupled to a proximal end portion of the needle, the needle driver comprising an actuation member, wherein the needle driver foreshortens from a first length to a second length upon movement of the actuation member for retracting a distal end portion of the needle from a first position to a second position relative to the housing.

Clause 10. The inserter of Clause 9, wherein the actuation member comprises an oblong cam, wherein the oblong cam is rotatable within the needle driver about an axis oriented perpendicular relative to a longitudinal axis of the inserter.

Clause 11. The inserter of Clause 10, wherein the oblong cam comprises a major axis and a minor axis, and the needle driver retracts from the first length to the second length by rotating the oblong cam to align the minor axis with the longitudinal axis of the inserter.

Clause 12. The inserter of any one of Clauses 9 to 11, wherein the actuation member positively displaces the needle driver from the first length to the second length.

Clause 13. The inserter of any one of Clauses 9 to 12, further comprising a removable key, wherein the removable key is releasably engaged with the actuation member to move the actuation member.

Clause 14. The inserter of Clause 13, wherein the removable key is releasably engaged with the actuation member to prevent release of a shunt.

Clause 15. The inserter of any one of Clauses 9 to 14, wherein the first position of the needle corresponds to an ab externo treatment.

Clause 16. The inserter of any one of Clauses 9 to 15, wherein the second position of the needle corresponds to an ab interno treatment.

Clause 17. The inserter of any one of Clauses 9 to 16, further comprising a needle base coupled to the proximal end portion of the needle, wherein the needle base is coupled to the needle driver.

Clause 18. An intraocular shunt inserter for treating glaucoma, comprising: a housing; a needle assembly at least partially disposed within the housing and comprising a needle and a needle driver coupled to a proximal end portion of the needle; a plunger assembly at least partially disposed within the housing and comprising a plunger and a plunger driver coupled to a proximal end portion of the plunger, the plunger being at least partially disposed within a lumen of the needle, the plunger being configured to advance a shunt through the lumen of the needle; and an actuation mechanism being rotatable to change at least one of a length of the needle driver or a length of the plunger driver for permitting a clinician to adapt the inserter for a given surgical procedure.

Clause 19. The inserter of Clause 18, wherein the plunger driver moves in an opposing direction to the needle driver upon movement of the actuation mechanism.

Clause 20. The inserter of Clause 19, wherein the plunger driver extends and the needle driver retracts upon movement of the actuation mechanism.

Clause 21. The inserter of any one of Clauses 18 to 20, wherein the actuation mechanism positively displaces the needle driver from a first length to a second length.

Clause 22. The inserter of any one of Clauses 18 to 21, further comprising a needle base coupled to the proximal end portion of the needle, wherein the needle base is coupled to the needle driver.

Clause 23. The inserter of any one of Clauses 18 to 22, wherein the actuation mechanism positively displaces the plunger driver from a first length to a second length.

Clause 24. The inserter of any one of Clauses 18 to 23, further comprising a removable key, wherein the removable key is releasably engaged with actuation mechanism to simultaneously move the plunger driver and the needle driver.

Clause 25. The inserter of Clause 24, wherein the removable key is releasably engaged with the actuation mechanism.

Clause 26. The inserter of any one of Clauses 18 to 25, wherein the plunger driver is at least partially nested within the needle driver.

Clause 27. An intraocular shunt inserter for treating glaucoma, comprising: a needle defining a lumen and comprising a needle driver coupled to a proximal end portion of the needle; a plunger assembly comprising a plunger at least partially disposed within the lumen of the needle, wherein the plunger is configured to advance a shunt through the lumen of the needle upon release; and a component driver comprising an actuation member, wherein the component driver elongates from a first length to a second length upon movement of the actuation member for changing a longitudinal relative position of (i) a distal end portion of the plunger, when coupled to a proximal end portion of the plunger, or (ii) a distal end portion of the needle, when coupled to a proximal end portion of the needle.

Clause 28. The inserter of Clause 27, wherein the actuation member comprises an oblong cam, wherein the oblong cam is rotatable within a body of the component driver about an axis oriented perpendicular relative to a longitudinal axis of the inserter.

Clause 29. The inserter of Clause 28, wherein the oblong cam comprises a major axis and a minor axis, and the component driver elongates from the first length to the second length by rotating the oblong cam to align the major axis with the longitudinal axis of the inserter.

Clause 30. The inserter of any one of Clauses 27 to 29, further comprising a removable key, wherein the removable key is releasably engaged with the actuation member to move the actuation member.

Clause 31. The inserter of any one of Clauses 27 to 30, wherein the removable key is releasably engageable with the actuation member.

Clause 32. The inserter of any one of Clauses 27 to 31, further comprising a housing, the needle or the plunger being longitudinally movable from a first position to a second position relative to a distal portion of the housing.

Clause 33. The inserter of any one of Clauses 27 to 32, further comprising any of the features recited in Clauses 1 to 26.

Clause 34. The inserter of any one of Clauses 27 to 33, wherein the component driver is coupled to the proximal end portion of the needle.

Clause 35. The inserter of any one of Clauses 27 to 34, wherein the component driver is coupled to the proximal end portion of the plunger.

Clause 36. A method of operating an intraocular shunt inserter, the method comprising: partially disposing a plunger within a lumen of a needle; and moving a plunger driver actuation member to change a plunger driver from a first plunger driver length to a second plunger driver length, wherein the plunger driver is coupled to a proximal end portion of the plunger.

Clause 37. The method of Clause 36, further comprising moving a distal end portion of the plunger from a first plunger position to a second plunger position relative to the lumen prior to releasing a shunt through the lumen of the needle.

Clause 38. The method of any one of Clauses 36 to 37, further comprising moving a needle driver actuation member to change a needle driver from a first needle driver length to a second needle driver length, wherein the needle driver is coupled to the proximal end portion of the plunger.

Clause 39. The method of Clause 38, further comprising moving a distal end portion of the needle from a first needle position to a second needle position relative to the distal end portion of the plunger prior to releasing a shunt through the lumen of the needle.

Clause 40. The method of any one of Clauses 38 to 39, further comprising moving the plunger driver actuation member and the needle driver actuation member simultaneously.

Clause 41. The method of any one of Clauses 38 to 40, further comprising moving the plunger driver in an opposing direction to the needle driver upon movement of the plunger driver actuation member and the needle driver actuation member.

Clause 42. The method of any one of Clauses 38 to 41, further comprising extending the plunger driver upon movement of the plunger driver actuation member; and retracting the needle driver upon movement of the needle driver actuation member.

Clause 43. The method of any one of Clauses 38 to 42, wherein moving the needle driver actuation member further comprises rotating an oblong cam within the needle driver about an axis oriented perpendicular relative to a longitudinal axis of the inserter.

Clause 44. The method of Clause 43, further comprising rotating the oblong cam to align a minor axis with the longitudinal axis of the inserter.

Clause 45. The method of any one of Clauses 38 to 44, further comprising positively displacing the needle driver from the first length to the second length.

Clause 46. The method of any one of Clauses 38 to 45, further comprising releasably engaging the plunger driver actuation member and the needle driver actuation member with a removable key.

Clause 47. The method of Clause 46, further comprising preventing release of a shunt by engaging the plunger driver actuation member and the needle driver actuation member with the removable key.

Clause 48. The method of any one of Clauses 36 to 47, wherein moving the plunger driver actuation member further comprises rotating an oblong cam within the plunger driver about an axis oriented perpendicular relative to a longitudinal axis of the inserter.

Clause 49. The method of Clause 48, further comprising rotating the oblong cam to align a major axis with the longitudinal axis of the inserter.

Clause 50. The method of any one of Clauses 36 to 49, further comprising positively displacing the plunger driver from the first length to the second length.

Clause 51. A method of operating an intraocular shunt inserter that incorporates any of the steps or features recited in any of Clauses 1 to 50.

Clause 52. An intraocular shunt inserter comprising any of the features recited in any of Clauses 1 to 50.

### Further Considerations

In some embodiments, any of the clauses herein may depend from any one of the independent clauses or any one of the dependent clauses. In one aspect, any of the clauses (e.g., dependent or independent clauses) may be combined with any other one or more clauses (e.g., dependent or independent clauses). In one aspect, a claim may include some or all of the words (e.g., steps, operations, means or components) recited in a clause, a sentence, a phrase or a paragraph. In one aspect, a claim may include some or all of the words recited in one or more clauses, sentences, phrases or paragraphs. In one aspect, some of the words in each of the clauses, sentences, phrases or paragraphs may be removed. In one aspect, additional words or elements may be added to a clause, a sentence, a phrase or a paragraph. In one aspect, the subject technology may be implemented without utilizing some of the components, elements, functions or operations described herein. In one aspect, the subject technology may be implemented utilizing additional components, elements, functions or operations.

A reference to an element in the singular is not intended to mean one and only one unless specifically so stated, but rather one or more. For example, "a" module may refer to one or more modules. An element proceeded by "a," "an," "the," or "said" does not, without further constraints, preclude the existence of additional same elements.

Headings and subheadings, if any, are used for convenience only and do not limit the invention. The word exemplary is used to mean serving as an example or illustration. To the extent that the term include, have, or the like is used, such term is intended to be inclusive in a manner similar to the term comprise as comprise is interpreted when employed as a transitional word in a claim. Relational terms such as first and second and the like may be used to distinguish one entity or action from another without necessarily requiring or implying any actual such relationship or order between such entities or actions.

Phrases such as an aspect, the aspect, another aspect, some aspects, one or more aspects, an implementation, the implementation, another implementation, some implementations, one or more implementations, an embodiment, the embodiment, another embodiment, some embodiments, one or more embodiments, a configuration, the configuration, another configuration, some configurations, one or more configurations, the subject technology, the disclosure, the present disclosure, other variations thereof and alike are for convenience and do not imply that a disclosure relating to such phrase(s) is essential to the subject technology or that such disclosure applies to all configurations of the subject technology. A disclosure relating to such phrase(s) may apply to all configurations, or one or more configurations. A disclosure relating to such phrase(s) may provide one or more examples. A phrase such as an aspect or some aspects may refer to one or more aspects and vice versa, and this applies similarly to other foregoing phrases.

A phrase "at least one of" preceding a series of items, with the terms "and" or "or" to separate any of the items, modifies the list as a whole, rather than each member of the list. The phrase "at least one of" does not require selection of at least one item; rather, the phrase allows a meaning that includes at least one of any one of the items, and/or at least one of any combination of the items, and/or at least one of each of the items. By way of example, each of the phrases "at least one of A, B, and C" or "at least one of A, B, or C" refers to only A, only B, or only C; any combination of A, B, and C; and/or at least one of each of A, B, and C.

It is understood that the specific order or hierarchy of steps, operations, or processes disclosed is an illustration of exemplary approaches. Unless explicitly stated otherwise, it is understood that the specific order or hierarchy of steps, operations, or processes may be performed in different order. Some of the steps, operations, or processes may be performed simultaneously. The accompanying method claims, if any, present elements of the various steps, operations or processes in a sample order, and are not meant to be limited to the specific order or hierarchy presented. These may be performed in serial, linearly, in parallel or in different order. It should be understood that the described instructions, operations, and systems can generally be integrated together in a single software/hardware product or packaged into multiple software/hardware products.

In one aspect, a term coupled or the like may refer to being directly coupled. In another aspect, a term coupled or the like may refer to being indirectly coupled.

Terms such as top, bottom, front, rear, side, horizontal, vertical, and the like refer to an arbitrary frame of reference, rather than to the ordinary gravitational frame of reference. Thus, such a term may extend upwardly, downwardly, diagonally, or horizontally in a gravitational frame of reference.

The disclosure is provided to enable any person skilled in the art to practice the various aspects described herein. In some instances, well-known structures and components are shown in block diagram form in order to avoid obscuring the concepts of the subject technology. The disclosure provides various examples of the subject technology, and the subject technology is not limited to these examples. Various modifications to these aspects will be readily apparent to those skilled in the art, and the principles described herein may be applied to other aspects.

All structural and functional equivalents to the elements of the various aspects described throughout the disclosure that are known or later come to be known to those of ordinary skill in the art are expressly incorporated herein by reference and are intended to be encompassed by the claims. Moreover, nothing disclosed herein is intended to be dedicated to the public regardless of whether such disclosure is explicitly recited in the claims. No claim element is to be construed under the provisions of 35 U.S.C. §112, sixth paragraph, unless the element is expressly recited using the phrase "means for" or, in the case of a method claim, the element is recited using the phrase "step for."

The title, background, brief description of the drawings, abstract, and drawings are hereby incorporated into the disclosure and are provided as illustrative examples of the disclosure, not as restrictive descriptions. It is submitted with the understanding that they will not be used to limit the scope or meaning of the claims. In addition, in the detailed description, it can be seen that the description provides illustrative examples and the various features are grouped together in various implementations for the purpose of streamlining the disclosure. The method of disclosure is not to be interpreted as reflecting an intention that the claimed subject matter requires more features than are expressly recited in each claim. Rather, as the claims reflect, inventive subject matter lies in less than all features of a single disclosed configuration or operation. The claims are hereby incorporated into the detailed description, with each claim standing on its own as a separately claimed subject matter.

The claims are not intended to be limited to the aspects described herein, but are to be accorded the full scope consistent with the language claims and to encompass all legal equivalents. Notwithstanding, none of the claims are intended to embrace subject matter that fails to satisfy the requirements of the applicable patent law, nor should they be interpreted in such a way.

The following numbered items also form part of the present disclosure.

Item 1: An intraocular shunt inserter for treating glaucoma comprises a housing; a needle assembly at least partially disposed within the housing and comprising a needle and a needle driver coupled to a proximal end portion of the needle; a plunger assembly at least partially disposed within the housing and comprising a plunger and a plunger driver coupled to a proximal end portion of the plunger, the plunger being at least partially disposed within a lumen of the needle, the plunger being configured to advance a shunt through the lumen of the needle; and an actuation mechanism being rotatable to change at least one of a length of the needle driver or a length of the plunger driver for permitting a clinician to adapt the inserter for a given surgical procedure.

Item 2: The inserter of Item 1, wherein the plunger driver moves in an opposing direction to the needle driver upon movement of the actuation mechanism.

Item 3: The inserter of Item 1 or 2, wherein the plunger driver extends and the needle driver retracts upon movement of the actuation mechanism.

Item 4: The inserter of any of Items 1 to 3, wherein the actuation mechanism comprises an oblong cam, wherein the oblong cam is rotatable within a body of the needle driver about an axis oriented perpendicular relative to a longitudinal axis of the inserter.

Item 5: The inserter of any of Items 1 to 4, wherein the actuation mechanism comprises an oblong cam, wherein the oblong cam is rotatable within a body of the plunger driver about an axis oriented perpendicular relative to a longitudinal axis of the inserter.

Item 6: The inserter of any of Items 1 to 5, wherein the actuation mechanism comprises an oblong cam that comprises a major axis and a minor axis, and wherein the length of the needle driver and the length of the plunger driver changes by rotating the oblong cam to align the major axis with the longitudinal axis of the inserter.

Item 7: The inserter of any of Items 1 to 6, further comprising a removable key, wherein the removable key is releasably engaged with the actuation member to move the actuation member.

Item 8: An intraocular shunt inserter for treating glaucoma, comprises a needle defining a lumen and a proximal end portion of the needle; a plunger assembly comprising a plunger at least partially disposed within the lumen of the needle, wherein the plunger is configured to advance a shunt through the lumen of the needle upon release; and a component driver comprising an actuation member, wherein the component driver elongates from a first length to a second length upon movement of the actuation member for changing a longitudinal relative position of (i) a distal end portion of the plunger, when coupled to a proximal end portion of the plunger, or (ii) a distal end portion of the needle, when coupled to a proximal end portion of the needle.

Item 9: The inserter of Item 8, wherein the actuation member comprises an oblong cam, wherein the oblong cam is rotatable within a body of the component driver about an axis oriented perpendicular relative to a longitudinal axis of the inserter.

Item 10: The inserter of Item 9, wherein the oblong cam comprises a major axis and a minor axis, and the component driver elongates from the first length to the second length by rotating the oblong cam to align the major axis with the longitudinal axis of the inserter.

Item 11: The inserter of any of Items 8 to 10, further comprising a removable key, wherein the removable key is releasably engaged with the actuation member to move the actuation member.

Item 12: The inserter of Item 11, wherein the removable key is releasably engageable with the actuation member.

Item 13: The inserter of any of Items 8 to 12, further comprising a housing, the needle or the plunger being longitudinally movable from a first position to a second position relative to a distal portion of the housing.

Item 14: A method of operating an intraocular shunt inserter comprises partially disposing a plunger within a lumen of a needle; and moving a plunger driver actuation member to change a plunger driver from a first plunger driver length to a second plunger driver length, wherein the plunger driver is coupled to a proximal end portion of the plunger.

Item 15: The method of Item 14, further comprising moving a distal end portion of the plunger from a first plunger position to a second plunger position relative to the lumen prior to releasing a shunt through the lumen of the needle.

Item 16: The method of any of Items 14 to 15, further comprising moving a needle driver actuation member to change a needle driver from a first needle driver length to a second needle driver length, wherein the needle driver is coupled to the proximal end portion of the plunger.

Item 17: The method of Item 16, further comprising moving a distal end portion of the needle from a first needle position to a second needle position relative to the distal end portion of the plunger prior to releasing a shunt through the lumen of the needle.

Item 18: The method of any of Items 16 to 17, further comprising moving the plunger driver actuation member and the needle driver actuation member simultaneously.

Item 19: The method of any of Items 16 to 18, further comprising moving the plunger driver in an opposing direction to the needle driver upon movement of the plunger driver actuation member and the needle driver actuation member.

Item 20: The method of any of Items 16 to 19, further comprising releasably engaging the plunger driver actuation member and the needle driver actuation member with a removable key.

## Claims

1. An intraocular shunt inserter for treating glaucoma, comprising:
a needle defining a lumen and a proximal end portion of the needle;
a plunger assembly comprising a plunger at least partially disposed within the lumen of the needle, wherein the plunger is configured to advance a shunt through the lumen of the needle upon release; and
a component driver comprising an actuation member, wherein the component driver elongates from a first length to a second length upon movement of the actuation member for changing a longitudinal relative position of (i) a distal end portion of the plunger, when coupled to a proximal end portion of the plunger, or (ii) a distal end portion of the needle, when coupled to a proximal end portion of the needle.

2. The inserter of Claim 1, wherein the actuation member comprises an oblong cam, wherein the oblong cam is rotatable within a body of the component driver about an axis oriented perpendicular relative to a longitudinal axis of the inserter.

3. The inserter of Claim 2, wherein the oblong cam comprises a major axis and a minor axis, and the component driver elongates from the first length to the second length by rotating the oblong cam to align the major axis with the longitudinal axis of the inserter.

4. The inserter of any one of Claims 1 to 3, further comprising a removable key, wherein the removable key is releasably engaged with the actuation member to move the actuation member.

5. The inserter of Claim 4, wherein the removable key is releasably engageable with the actuation member.

6. The inserter of any one of Claims 1 to 5, further comprising a housing, the needle or the plunger being longitudinally movable from a first position to a second position relative to a distal portion of the housing.
